# EUROPÄISCHE PATENTSCHRIFT

(11) **EP 2 730 249 B1**
(45) Veröffentlichungstag und Bekanntmachung des Hinweises auf die Patenterteilung: **15.04.2015**
(21) Anmeldenummer: 13187915.7
(22) Anmeldetag: 09.10.2013
(51) Int. Cl.: A61B 19/08, A61B 19/02, A61B 19/00, A61B 5/00, A61B 17/00, A47B 21/00, A47B 21/03

(54) **Stationswagen, insbesondere Visiten- und Pflegewagen**
Trolley, in particular ward trolley and care trolley
Chariot, notamment chariot de visites et de soins

(30) Priorität: 09.11.2012 DE 202012104320 U; 09.11.2012 DE 202012104323 U; 09.11.2012 DE 202012104316 U
(43) Veröffentlichungstag der Anmeldung: 14.05.2014
(60) Teilanmeldung: 14183925.8
(73) Patentinhaber: Optiplan Gesellschaft für optische Planungsgeräte mit beschränkter Haftung, 40489 Düsseldorf (DE)
(72) Erfinder: Wagner, Kai, 40489 Düsseldorf (DE); Lerner, Sabine, 40489 Düsseldorf (DE)
(74) Vertreter: Stenger, Watzke & Ring

(56) Entgegenhaltungen:
- WO-A1-98/02107
- DE-A1- 19 747 353
- DE-A1-102009 036 941
- DE-U1-202010 005 903
- DE-U1-202011 051 973
- DE-U1-202012 101 860
- FR-A1- 2 652 247
- US-A- 5 655 743
- US-A- 5 765 565
- US-A1- 2004 262 867
- US-A1- 2007 227 409
- US-A1- 2009 125 014

## Beschreibung

Die Erfindung betrifft einen Stationswagen, insbesondere einen Visiten- und Pflegewagen für Krankenhäuser, Pflegeheim und sonstige Einrichtungen des Gesundheitswesens, mit einem verfahrbar ausgebildeten Schrankteil, das der Aufnahme unter anderem einer Rechneranlage dient, und mit einem Monitor, der verdrehbar an einem Tragrohr angebracht ist.

Stationswagen im Allgemeinen sowie solche mit einem Monitor im Speziellen sind aus dem Stand der Technik gut bekannt. So sind Stationswagen beispielsweise aus der DE 297 14 981, der DE 20 2005 013 115, der DE 20 2005 013 114 der DE 20 2008 015 373 und der DE 20 2011 051 973 bekannt geworden.

Auch hinsichtlich des mit einem vorbekannten Stationswagen zum Einsatz kommenden Monitors sind aus dem Stand der Technik unterschiedliche Monitorbauformen bekannt geworden. Eine insbesondere in letzter Zeit häufig eingesetzte Bauförm betrifft Monitore mit einem sogenannten Touchscreen. Gemäß dieser Monitorbauform findet nicht nur eine Anzeige über den Monitor statt, es wird zugleich auch eine Eingabemöglichkeit bereitgestellt. Diese erfolgt zumeist über eine Berührung des Monitorbildschirms, die geräteseitig sensiert und mittels einer entsprechenden Software erkannt und verarbeitet wird. Da ein Touchscreen gleichermaßen als Anzeige- und Eingabemittel dient, kann in vorteilhafter Weise auf ergänzende Peripheriegeräte zur Eingabe wie zum Bespiel Tastaturen, Mäuse und/oder dergleichen verzichtet werden.

Monitore mit Touchscreen-Funktion haben sich im. allfäglichen Praxiseinsatz bewährt. In Krankenhäusern, Pflegeheimen und sonstigen Einrichtungen des Gesundheitswesens ist aus hygienischen Gründen der Einsatz von Monitoren mit Touchscreen-Funktion allerdings nicht überall möglich. Insbesondere in Bereichen mit Patientenkontakt ist der Einsatz aus dem Stand der Technik bekannter Monitore mit Touchscreen-Funktion bedenklich, da die für eine bestimmungsgemäße Verwendung notwendig Bildschirmberührung die Gefahr mit sich bringt, dass Erreger, Keime, Bakterien und/oder dergleichen biologische Materialien ungewollt übertragen und damit verbreitet werden. Ob der Empfindlichkeit vorbekannter Touchscreens kommt auch eine Reinigung der Benutzeroberfläche mit den im Gesundheitswesen standardmäßig verwendeten Reinigungsmitteln nicht in Frage. Insbesondere solche Reinigungsmittel, die der Abtötung von biologischem Material dienen, können die empfindliche Bildschirmoberfläche von Monitoren mit Touchscreen-Funktion zerstören und/oder unbrauchbar machen.

Die DE 197 47 353 A1 beschreibt in diesem Zusammenhang ein Patiententerminal mit integriertem Display. Das Terminal verfügt ferner über eine hygienische Glasfront mit integrierten Tuuch-Screen-Funktionen zur direkt kontrollierbaren, softwaregestützten, Steuerung des Systems und zur kontrollierten Eingabe der Leistungserfassung. Das Gehäuse hat durch seine Touch-Screen-Glasfront keine hervorstehenden Tasten und Schalter. Es ist daher desinfektionsmittetfest und spritzwasserdicht und insofern gut zu reinigen. Angebracht kann besagtes Patiententerminal mittels eines schwenkbaren Arms an einem Nachttischwagen sein. Nachteile ergeben sich insbesondere daraus, dass in ihrer Mobilität und/oder ihrer Größe eingeschränkte Personen während einer ausgeschwenkten stellung des Armes keinen oder lediglich einen eingeschränkten Zugang zum Nachttischwagen haben. Die Offenlegungsschrift DE 197 47 353 A1 offenbart der Gegenstand der Präambel von Anspruch 1.

Es ist deshalb ausgehend vom vorbeschriebenen Stand der Technik die **Aufgabe** der Erfindung, einen Stationswagen mit einem Monitor bereitzustellen, der verbessert ist.

Zur **Lösung** dieser Aufgabe wird mit der Erfindung ein Stationswagen gemäß Anspruch 1 vorgeschlagen. Weiterbildungen sind in der abhängiger Ansprüchen angegeben.

Der Monitor nach der Erfindung verfügt über einen aus dem Stand der Technik an sich bekannten, standardmäßigen Touchscreen. Dieser ist, erfindungsgemäß vollständig gekapselt ausgebildet, was einen Schutz vor äußeren Einflüssen, insbesondere ungewollten Verschmutzungen bietet. Frontseitig des Touchscreens ist zudem eine Abdeckplaltte aus einem transparenten Material angeordnet. Diese Abdeckplatte ist - anders als die Bildschirmoberfläche des Touchscreens - gegenüber herkömmlichen Reinigungsmitteln unempfindlich, so dass eine auch den in einem Krankenhaus, einem Pflegeheim und/oder einer sonstigen Einrichtung des Gesundheitswesens geltenden Vorschriften entsprechende Reinigung durchgeführt werden kann. Die erfindungsgemäße Ausgesteltung gestattet es mithin, Monitore mit Touchscreen-Funktion einzusetzen und die damit einhergehenden Vorteile zu nutzen, andererseits aber insbesondere aus hygienischen Gründen sicherzustellen, dass eine vörschriffsmäßige Reinigung erfolgen kann, ohne dass die Gefahr einer durch die Reinigung bedingten Funktionsbeeinträchtigung besteht.

Der nach der Erfindung vorgeschlagene Monitor eignet sich damit insbesondere auch zur Verwendung im Stationsbereich, beispielsweise eines Krankenhauses, d. h. im Bereich der direkten Patientenbetreuung und/oder -pflege Dank des vorgesehenen Touchscreens kann eine Eingabe ohne zusätzliche Peripheriegeräte wie beispielsweise eine Tastatur erfolgen. Gleichzeitig ist Dank der gekapselten Ausgestaltung sichergestellt, dass ein Schutz vor ungewollten Verschmutzungen gegeben ist, wobei im Verschmutzungsfall eine die Funktion des Monitors nicht beeinträchtigende Reinigung stattfinden kann. Durch die frontseitig vorgesehene Abdeckplatte erfolgt insbesondere ein Schutz des auch als Bedienoberfläche dienenden Bildschirms, welche Abdeckplatte aus einem transparenten Material gebildet ist, so dass die Funktionstüchtigkeit des Touchscreens in vollem Umfang gegeben ist. Im Verschmutzungsfall erfolgt eine frontseitige Reinigung der Abdeckplatte, die hinsichtlich ihrer Materialwahl derart ausgebildet ist, dass eine Reinigung mit im Gesundheitswesen standardmäßig verwendeten Reinigungsmitteln erfolgen kann.

Die Abdeckplatte besteht aus Glas oder aus Kunststoff. Es ist indes bevorzugt, als Abdeckplatte eine Glasscheibe oder ein Glaspaneel zu verwenden. Insbesondere aus reinigungstechnischen Gründen hat sich der Einsatz von Glas bewährt. Darüber hinaus ist Glas weniger kratzanfällig und alterungsbeständiger äls Kunststoff.

Gemäß der Erfindung verfügt das Schrankteil des Stationswagens über ein Schrankunterteil einerseits und ein Schrankoberteil andererseits. Dabei dient das Schrakunterteil der Aufnahme der Rechneranlage.

Das Schrankoberteil ist in Höhenrichtung oberhalb des Schrankunterteils angeordnet. Es ist in Höhenrichtung relativ verfahrbar zum Schrankunterteil ausgebildet, so dass eine individuelle Höheneinstellung des Schrankoberteils benutzerseitig eingestellt werden kann.

Das Schrankobertel ist von einer Säule getragen. Dabei ist erfindungsgemäß vorgesehen, dass es sich bei der Säule um eine teleskopierbare Säule handelt. Eine Höhenverstellung erfolgt demnach durch eine Teleskopierbewegung der Säule. Auf einfache Weise ist es einem Benutzer des Stationswagens mithin gestattet, eine auf den individuellen Anwendungsfall bezogene Höheneinstellung des Schrankoberteils in Relation zum Schrankunterteil vornehmen zu können.

Die Säule ist mit ihrem dem Schrankoberteil gegenüberliegenden Endabschnitt am Schrankunterteil angeordnet. Insofern bilden das Schrankunterteil, die Säule und das Schrankoberteil eine gemeinsame Einheit. Diese ist verfahrbar ausgebildet, was eine freie Positionierung im Raum gestattet.

Die teleskopierbare Saule weist erfindungsgemäß zwei Einzelrohre auf. Diese Einzelrohre sind jeweils teleskopierbar ausgebildet und im endmontierten Zustand einander nebengeordnet. Diese Ausgestaltung erweist sich insbesondere aus Stabilitätsgründen von Vorteil. Anstelle eines Einzelrohres kommt nach der Erfindung eine Säule zum Einsatz, die nach Art eines Doppelrohres aus zwei Einzelrohren gebildet ist. Eine verbesserte Steifgkeit insbesondere gegenüber Biegebeanspruchungen wird so erreicht. Dies erweist sich insbesondere bei vollbeladenem Schrankoberteil von Vorteil.

Die die Säule bildenden Einzelrohre stellen jeweils einen inneren Volumenraum bereit. Dabei ist es erfindungsgemäß vorgesehen, dass das eine der beiden Einzelrohre als Kabelkanal und das andere der beiden Einzelrohre der Aufnahme eines elektromotorischen Antriebs dient.

Der für eine Höhenverstellung erfindungsgemäß vorgesehene elektromotorische Antrieb kann beispielsweise als Spindelantrieb ausgebildet sein. Er ist erfindungsgemäßer Weise gekapselt innerhalb des einen Einzelrohres angeordnet, womit eine vor äußeren Einflüssen weitestgehend geschützte Anordnung erreicht ist, was in vorteilhafter Weise zu einer erhöhten Lebensdauer führt.

Das andere der beiden Einzelrohre dient erfindungsgemäß als Kabelkanal. Im endmontierten Zustand sind durch dieses beispielsweise eine Rechnerverkabelung geführt, insbesondere eine einendseitig mit dem Monitor des Stationswagens verbundene Verkabelung.

Für eine ergänzende Versteifung können die die Säule bildenden Einzelrohre miteinander mechanisch gekoppelt, d.h. verbunden sein. Zu diesem Zweck kommen bevorzugter Weise Verbinder zum Einsatz, die mit den Einzelrohren verschraubt sind. Es wird so eine kompakte Baueinheit geschaffen.

Ein jedes der beiden Einzelrohre verfügt bevorzugter Weise über drei Segmente, die teleskopierbar verfahrbar ausgebildet sind. Dabei kann ein unteres mit dem Schrankunterteil gekoppeltes erstes Segment, ein oberes, mit dem Schrankoberteil gekoppeltes zweites Segment sowie ein diese beiden Segmente miteinander verbindendes mittleres, drittes Segment vorgesehen sein. Das obere Segment ist dabei innerhalb des mittleren Segments und das mittlere Segment innerhalb des unteren Segments verfahrbar geführt. Dabei ist es bevorzugt, die beiden Einzelrohre identisch zueinander auszubilden.

Der Wagen dient der Aufnahme von elektrischen Geräten, insbesondere zur Aufnahme eines Rechners. In diesem Fall ist das Schrankunterteil für die Aufnahme des eigentlichen Rechners vorgesehen. Das Schrankoberteil dient indes als Ablagefläche und insbesondere auch der Aufnahme von Rechnerbedieneinheiten, wie z. B. einer Tastatur, einer Maus und/oder dergleichen Peripheriegeräte. Zum Zwecke der elektrischen und/oder elektronischen Verbindung der einzelnen Bestandteile bzw. Komponenten einer Rechneranlage ist ein Kabelkanal vorgesehen, der bevorzugterweise im Inneren der Säule verlaufend ausgebildet ist. In vorteilhafter Weise kann so eine völlig versteckte Anordnung von Kabeln erfolgen, was einerseits aus hygienischen Gründen und andererseits aus sicherheitsrelevanten Gründen von Vorteil ist.

Das Schrankoberteil ist mit einem Monitorhalter in Form des Tragrohrs ausgerüstet. Dabei ist bevorzugterweise vorgesehen, dass der Monitorhalter eine relativ zum Schrankoberteil verschwenkbare Anordnung eines Monitors gestattet. Es ist mithin eine Ausrichtung des Monitors relativ gegenüber dem Schrankoberteil und damit relativ gegenüber dem gesamten Wagen möglich. In Kombination mit der Höhenverstellmöglichkeit des den Monitorhalter tragenden Schrankoberteils kann verwenderseitig eine individuellen Wünschen gerecht werdende Ausrichtung des Monitorhalters bzw. des davon getragenen Monitors vorgenommen werden.

Eine. Bedienung des Stationswagens zwecks Höhenverstefiung sowie gegebenenfalls einer Ausrichtung des Monitors erfolgt bevorzugterweise über die Touchscreen-Oberfläche des Monitors. Bevorzugterweise kommt zu diesem Zweck ein Touchscreen in Form einer Folienscheibe zum Einsatz, die zum Vorsehen derlei Bedienelemente die eigentliche Monitorfläche zumindest an einem Flächenrand überragt, wobei die gesamte Folienscheibe frontseitig durch die Abdeckplatte abgedeckt ist.

Es ist gemäß einem weiteren Merkmal der Erfindung vorgesehen, dass das Schrankoberteil einen nach Art einer Schublade aus dem Schrankoberteil heraus verfahrbaren Auszug bereitstellt. Dieser Auszug kann insbesondere zur Aufnahme einer Tastatur dienen. Im Verwendungsfall wird sie mit Hilfe des Auszugs aus dem Schrankoberteil heraus verfahren und ist für einen Verwender frei zugänglich. Im Nicht-Verwendungsfall kann sie platzsparend innerhalb des Schrankoberteils durch einfaches Einfahren des Auszuges angeordnet werden.

Der Auszug kann gemäß einem weiteren Merkmal der Erfindung seinerseits über ein quer zur Verfahrrichtung des Auszugs verfahrbares Plattenelement verfügen. Dabei ist es bevorzugt, dass die Verfahrrichtung von Auszug einerseits und Plattenelement andererseits orthogonal zueinander ausgerichtet sind.

Im Verwendungsfall kann Dank der vorbeschriebenen Konstruktion ein zweiteiliges Ausfahren stattfinden. In einem ersten Schritt kann zunächst der Auszug aus dem Schrankoberteil aufgefahren werden. Alsdann kann in einem zweiten Schritt das Plattenelement relativ zum Auszug verfahren werden, indem dieses in einer quer zur Verfahrrichtung des Auszuges liegenden Verfahrrichfung bewegt wird. Es ist dabei bevorzugt, dass das Plattenelement wahlweise zur einen oder zur anderen Seite des Auszugs verfahren werden kann, womit eine gleichermaßen bestimmungsgemäße Verwendung des Wagens sowohl für Linkshänder als auch für Rechtshänder stattfinden kann.

Der Monitor kann über eine Monitoreinheit verfügen. Bei dieser Monitoreinheit kann es sich um einen aus dem Stand der Technik an sich bekannten, standardmäßigen Monitor ohne Touchscreen-Funktion handeln. Dieser als Monitoreinheit dienende Monitor trägt frontseitig die Abdeckplatte. Zwischen der Monitoreinheit und der Abdeckplatte ist der Touchscreen angeordnet. In ihrer Kombination bilden die Monitoreinheit und der Touchscreen einen Monitor mit Touchscreen-Funktion. Die frontseitig des Touchscreens angeordnete Abdeckplatte dient sowohl dem Schutz des Touchscreens als auch der in Blickrichtung auf den Monitor dahinter angeordneten Monitoreinheit.

Als Touchscreen kommt eine kapazitive Folienscheibe zum Einsatz. Diese ist vorzugsweise rückseitig auf die Abdeckplatte aufgeklebt. Im Verwendungsfall wird bei einer benutzerseitigen Berührung der Abdeckplatte eine entsprechende Sensierung durch die Folienscheibe bewirkt, wobei in Entsprechung der Sensierung eine Signalabgabe an die Rechneranlage erfolgt. Hier findet dann in an sich bekannter Weise eine entsprechende Datenverarbeitung statt.

Der Einsatz einer Monitoreinheit einerseits und eines als separates' Bauteil ausgebildeten Touchscreens andererseits ermöglicht in der Kombination einen sandwichartigen Aufbau, der es gestattet, wahlweise in ihrer Bauform unterschiedliche Monitoreinheiten einzusetzen, je nach Anwendungsfall und/oder Kundenwunsch. Der rückseitig auf die Abdeckplatte aufgeklebt Touchscreen ermöglicht eine benutzerseitige Steuerung der Rechneranlage ohne Peripheriegeräte, wobei Dank der Abdeckplatte ein Schutz sowohl des Touchscreens als auch der Monitoreinheit gegeben ist, was insbesondere aus hygienischen Gründen von Vorteil ist und den Einsatz des mit einem solchen Monitor ausgerüstete Stationswagens auch im Bereich der direkten Patientenbetreuung und/oder -pflege gestattet.

Der Aufbau erbringt einen weiteren Vorteil. So ist es gestattet, die als Touchscreen dienende Folienscheibe größer als die frontseitige Fläche der Monitoreinheit auszubilden. In diesem Fall überragt die Folienscheibe die frontseitige Fläche der Monitoreinheit randseitig. Es ist so gestattet, außerhalb der frontseitigen Fläche der Monitoreinheit Bedieneinheiten vorzusehen, die Dank der frontseitig ausgebildeten Abdeckplatte ebenfalls vollständig geschützt sind. Derlei Bedieneinheiten können beispielsweise solche für eine Höhenverstellung des Stationswagens, für eine Verdrehbewegung des Monitors, für eine Ein- und Ausschaltung der Rechneranlage und/oder dergleichen sein. Bei aus dem Stand der Technik bekannten Stationswagen kommen zu diesem Zweck typischerweise Schalttasten oder Kippschalter zum Einsatz, die beispielsweise am Schrankteil des Stationswagens angeordnet sind. Mit der erfindungsgemäßen Ausgestaltung können derlei Schalter vollends entfallen. Sie sind in ihrer Funktion mittels der als Touchscreen dienenden Folienscheibe ausgebildet, was einerseits eine einheitliche und damit einfache und ergonomische Handhabung des Stationswagens gestattet, andererseits aber auch aus hygienischen Gründen von Vorteil ist, da unnötige Stellen, an denen sich ungewollt Schmutz, Bakterien und/oder dergleichen ansammeln können, entfallen und darüber hinaus auch eine vereinfachte Reinhaltung gestattet ist.

Der oder die Teilbereiche der Folienscheibe, die über die frontseitig Fläche der Monitoreinheit überstehen, d.h. die frontseitige Fläche randseitig überragen sind gemäß einem weiteren Merkmal der Erfindung mittels eines Rahmens abgestützt. Es ist zu diesem Zweck vorgesehen, dass die Monitoreinheit von einem bündig mit der frontseitigen Fläche der Monitoreinheit abschließenden Rahmen umgeben ist, der den die frontseitige Fläche der Monitoreinheit überragenden Teil der Folienscheibe abstützt. Dieser Rahmen ist bevorzugterweise unter Zwischenordnung der Folienscheibe auf die Abdeckplatte aufgeklebt. Dies sichert einen formstabilen Verbund, der den im alltäglichen Praxiseinsatz des Stationswagens auftretenden mechanischen Belastungen gerecht wird. Der Rahmen unterstützt zudem eine lagesichere Fixierung von Monitoreinheit und Abdeckplatte bzw. Touchscreen.

Es kann hierbei vorgesehen sein, dass das Gehäuse des Monitors rückseitig der Abdeckplatte anliegt. Dabei ist das Monitorgehäuse vollständig geschlossen ausgebildet und die Anordnung an der Abdeckplatte ist ebenfalls fugendicht ausgebildet. Dabei ist die Anbindung des Monitorgehäuses an der Abdeckplatte in der Außenkontur vorsprungsfrei, so dass in vorteilhafter Weise etwaige Ansammelstellen für Verschmutzungen, Verunreinigungen und/oder dergleichen vollends vermieden sind. Der gesamte Monitor ist mithin in der Zusammenwirkung von Monitorgehäuse einerseits und Abdeckplatte andererseits als vollgekapseltes Bauteil ausgebildet, so dass im Bedarfsfall eine Reinigung insbesondere auch mit in Krankenhäusern, Pflegeheimen und/oder dergleichen üblicherweise verwendeten Reinigungsmitteln erfolgen kann.

Es liegen auch keinerlei Kabel oder Kabelstränge offen. Das Tragrohr für den Monitor stellt einen Kabelkanal zur Verfügung. Dieser dient der Aufnahme von Kabeln und/oder eines mehrere Kabel umfassenden Kabelstrangs, die bzw. der vom Monitor abgehen. Dies können einerseits Stromversorgungskabel sowie andererseits Datenübertragungskabel sein.

Das Monitorgehäuse verfügt tragrohrseitig über eine Manschette. Diese Manschette deckt den monitorseitigen Endabschnitt des Tragrohres ab und damit insbesondere auch den Bereich der Einführung der Kabel in den vom Tragrohr bereitgestellten Kabelkanal. Freiliegende Kabel und/oder Kabelabschnitte sind so in vorteilhafter Weise vermieden, was aus den schon vorerlaüterten Hygienegründen von Vorteil ist.

Es kann alternativ auch eine Monitoreinheit zum. Einsatz kommen, die ihrerseits bereits über eine Touchscreen-Funktion verfügt. So kann die Monitoreinheit beispielsweise ein Touchscreen-Monitor sein. Gemäß dieser alternativen Ausgestaltung ist die Abdeckplatte unter Belassung eines Spalts beabstandet zum. Touchscreen angeordnet. Diese Ausgestaltung dient zum einen dem zusätzlichen Schutz der Bildschirmobefläche des Touchscreens. Zum anderen ist im Spalt zwischen Abdeckplatte und Touchscreen eine Eingabeübertragungseinheit angeordnet. Diese Eingabeübertragungseinheit dient dazu, im bestimmungsgemäßen Einsatzfäll durch Berührung der Abdeckplatte benutzerseitig getätigte Eingabebefehle auf die Bitdschirmoberfläche des Touchscreens zu übertragen. Die Zwischenschaltung der Eingabeübertragungseinheit gestattet es mithin in vorteilhafter Weise, einen standardmäßigen Touchscreen zu verwenden. Die benutzerseitig über die dem Touchscreen frontseitig vorgeschaltete Abdeckplatte durch Berührung eingegebenen Befehle werden 1 : 1 auf den Touchscreen übertragen, so dass unter Verwendung standardmäßiger Software eine Eingabeverarbeitung stattfinden kann.

Die Eingabeübertragungseinheit kann nach Art einer Folie ausgebildet sein. Sie kann innenseitig der Abdeckplatte mit dieser verklebt sein. Dies gestattet vorteilhafterweise eine Vormontage, so dass es für eine endfertige Herstellung eines erfindungsgemäßen Monitors lediglich erforderlich ist, einen Touchscreen mit einer entsprechend vorbereiteten Abdeckplatte auszurüsten und zwecks vollständiger Kapselung in einem entsprechend ausgebildeten Gehäuse anzuordnen.

Die Eingabeübertragungseinheif kann eine kapazitiv arbeitende Folienscheibe sein. Mittels dieser Folienscheibe können die abdeckplattenseitig eingegebenen Benutzerbefehle unmittelbar auf die Bildschirmoberfläche des Touchscreens übertragen werden. Zeitliche Verzögerungen in der Befehlsweiterleitung werden so ausgeschlossen. Alternativ kann anstelle einer kapazitiven Folienscheibe auch eine induktive Folienscheibe zum Einsatz kommen.

Insgesamt wird mit der erfindungsgemäßen Ausgestaltung ein Stationswagen vorgeschlagen, der über einen gegenüber der äußeren Umgebung vollständig geschützt ausgebildeten, mithin gekapselten Monitor verfügt. Der Monitor stellt keine Schlitze, Öffnungen oder dergleichen bereit, durch die hindurch Schmutz, Verunreinigungen und/oder dergleichen ins Innere des Monitors eindringen können. Auch andere Ansammelstellen für Verunreinigungen, Verschmutzungen und/oder dergleichen sind nicht vorhanden. Es kann eine Komplettreinigung auch mit flüssigen Reinigungsmitteln stattfinden. Es sind darüber hinaus keine freiliegenden Kabel, vorgesehen. Diese sind vom Monitorgehäuse geschützt über das den Monitor aufnehmende Tragrohr abgeführt und im Inneren des Stationswagens vor äußeren Eingriffen und Zugriffen geschützt bis zur vom Stationswagen bereitgestellten Rechneranlage verlegt.

Weitere Merkmale und Vorteile der Erfindung ergeben sich aus der nachfolgenden Beschreibung anhand der Figuren. Dabei zeigen:
- Fig. 1: in einer schematischen Perspektivansicht einen Wagen nach der Erfindung in der Ausgestaltung eines Pflegewagens für ein Krankenhaus;
- Fig. 2: in einer schematischen Perspektivansicht die Säule des Wagens nach Fig. 1 in einer Detaildarstellung;
- Fig. 3: in einer schematischen Perspektivansicht das Schrankoberteil des erfindungsgemäßen Wagens nach Fig. 1 in einer Detaildarstellung;
- Fig. 4: in einer schematisch teilgeschnittenen Seitenansicht einen Monitor gemäß einer ersten Ausführungsform und
- Fig. 5: in einer schematisch teilgeschnittenen Seitenansicht einen Monitor gemäß einer zweiten Ausführungsform.

Der erfindungsgemäße Stationswagen 1 verfügt über ein Schrankunterteil 2. Dieses Schrankunterteil 2 verfügt über Pfosten 8, die der Anordnung und gegenseitigen Abstützung von Seitenwänden dienen. Frontseitig verfügt das Schrankunterteil 2 über eine verschwenkbar ausgebildete Flügeftür 16.

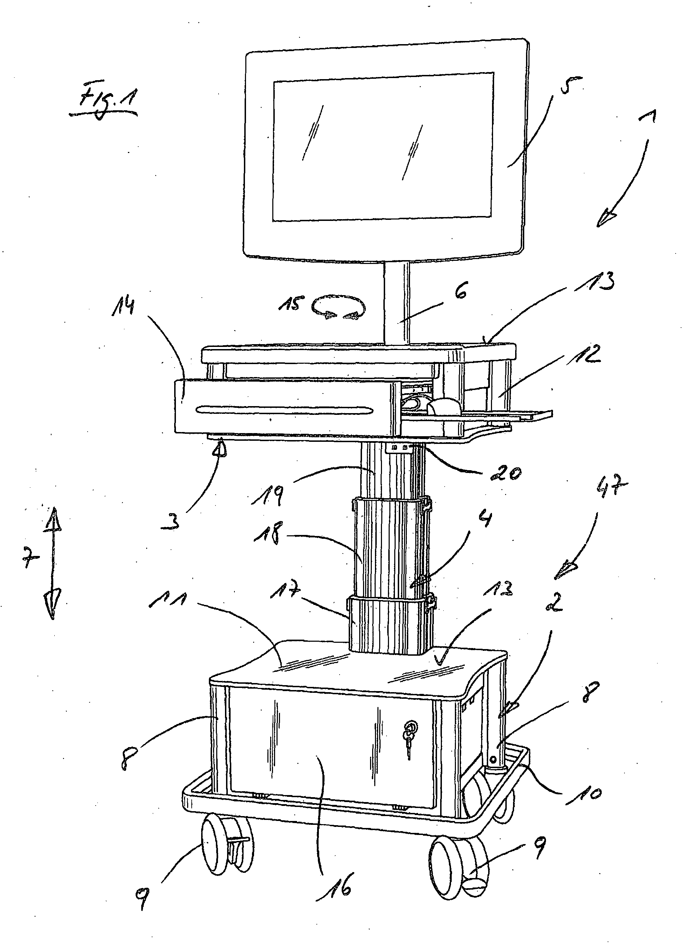

Die Pfosten 8 tragen fußbodenseitig Tragrollen 9, womit der Stationswagen 1 insgesamt verfahrbar ausgebildet ist. Das Schrankunterteil 2 kann zudem mit einem umlaufenden Rammschutz 10 ausgerüstet sein, wie das Ausführungsbeispiel nach Figur 1 erkennen lässt.

Oberseitig schließt das Schrankunterteil 2 mit einer Deckplatte 11 ab, die ihrerseits eine Ablagefläche 13 bereitstellt.

Der Stationswagen 1 verfügt des Weiteren über eine Säule 4. Diese ist in Höhenrichtung 7 verlaufend ausgerichtet und am Schrankunterteil 2 angeordnet.

Die Säule 4 trägt ein Schrankoberteil 3. Die Säule 4 ist teleskopierbar ausgebildet, womit das Schrankoberteil 3 in Längsrichtung der Säule 4, d.h. in Höhenrichtung 7 verfahrbar von der Säule 4 getragen. In der Konsequenz ist das Schrankoberteil 3 in Höhenrichtung 7 relativ verfahrbar zum Schrankunterteil 2 ausgebildet. Es kann also eine individuellen Wünschen gerecht werdende Ausrichtung des Schrankoberteils 3 in Höhenrichtung 7 relativ gegenüber dem Schrankunterteil 2 stattfinden.

Das Schrankoberteil 3 ist nach Art eines einseitig offenen Kastenteils 12 ausgebildet. Es stellt oberseitig eine Ablagefläche 13 bereit und verfügt über einen nach Art einer Schublade aus dem Schrankoberteil 3 heraus verfahrbaren Auszug 14. Dieser Auszug 14 kann insbesondere der Aufnahme einer Tastatur einer Rechneranlage dienen.

Das Schrankoberteil 3 ist des Weiteren mit einem Monitorhalter 6 ausgerüstet. Dieser Monitorhalter 6 trägt einen Monitor 5, wobei der Monitorhalter 6 den Monitor 5 in Pfeilrichtung 15 verschwenkbar aufnimmt. Gemäß der Ausführungsform nach Fig. 1 ist der Monitorhalter 6 mittig des Schrankoberteils 3 ausgebildet. Eine seitliche Ausgestaltung des Monitorhalters 6 ist selbstverständlich ebenfalls möglich.

Die Säule 4 nimmt einen im Säuleninneren verlaufenden Kabelkanal auf. Kabel, insbesondere elektrische Verbindungskabel können so versteckt geführt werden. Ferner nimmt die Säule 4 einen elektromotorischen Antrieb auf.

Es ist vorgesehen, dass die Säule 4 zwei Einzelrohre 21 und 22 aufweist. Diese Einzelrohre 21 und 22 sind jeweils teleskopierbar ausgebildet. Das eine der beiden Einzelrohre stellt dabei den Kabelkanal zur Verfügung, wohingegen das andere Einzelrohr der.Aufnahme eines elektromotorischen Antriebs dient.

Der Visiten- und Pflegewagen 1 dient unter anderem als Rechnerwagen, wobei das Schrankunterteil 2 die Rechnereinheit aufnimmt. Das Schrankoberteil 3 dient der Aufnahme von Bedieneinheiten, wie z. B. einer Tastatur oder einer Maus. Eine Kabelverbindung zwischen diesen Komponenten der Rechneranlage erfolgt über den Kabelkanal innerhalb der Säule 4. Die Verbindungskabel zum Monitor 5 werden gleichfalls versteckt geführt, und zwar über den Monitorhalter 6, das Schrankoberteil 3 und die Säule 4 bis hin zum Schrankunterteil 2.

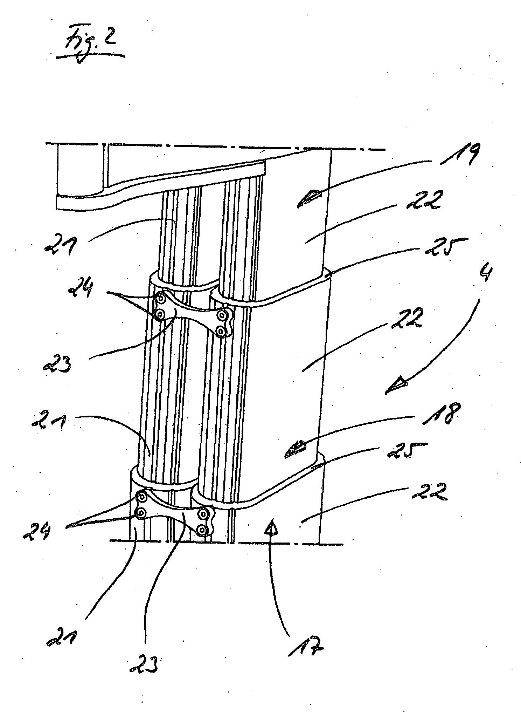

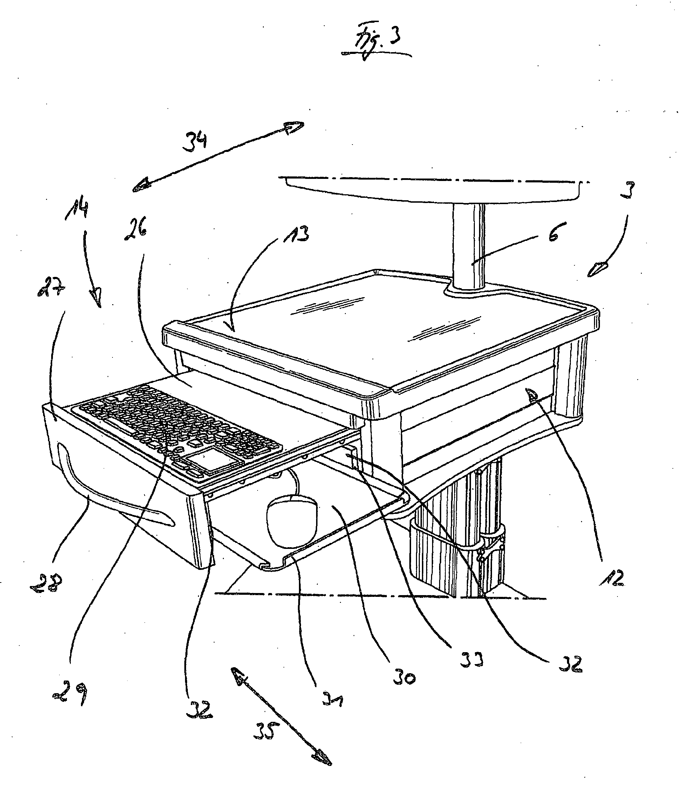

Wie sich insbesondere aus einer Zusammenschau der Figuren 2 und 3 ergibt, ist die das Schrankoberteil 3 tragende Säule 4 teleskopierbar ausgebildet und verfügt über insgesamt drei Segmente 17, 18 und 19, nämlich mit Bezug auf die Erstreckung des Stationswagen 1 in Höhenrichtung 7 über ein unteres Segment 17, ein mittleres Segment 18 und ein oberes Segment 19. Dabei ist das obere Segment 19 innerhalb des mittleren Segments 18 und das mittlere Segment 18 innerhalb des unteren Segments 17 in Höhenrichtung 7 verfahrbar.

Die Höhenverstellung erfolgt elektromotorisch. Es ist zu diesem Zweck ein entsprechender Antrieb vorgesehen, beispielsweise in Form eines Spindelantriebs. Dieser ist innerhalb der Säule 4 angeordnet. Zum Zwecke der Bedienung ist eine am oberen Segment 19 angeordnete Bedieneinheit 20 vorgesehen. Diese verfügt im einfachsten Fall über Bedienköpfe die verwenderseitig für eine Höhenverstellung der Säule 4 zu betätigen sind.

Die Säule 4 verfügt über ein erstes Rohr 21 und ein zweites Rohr 22. Diese Rohre 21 und 22 sind jeweils als Ovalrohre ausgebildet. Sie sind direkt zueinander benachbart ausgebildet und über entsprechende Verbinder 23 zu einer gemeinsamen Einheit gekoppelt. Als Verbinder 23 können - wie in Fig. 2 dargestellt - vergleichsweise flach ausgebildete Platten zum Einsatz kommen, die mit den Rohren 21 und 22 über entsprechende Schrauben 24 jeweils verschraubt sind.

Die beiden Rohre 21 und 22 bilden zusammen ein "Doppelrohr" aus, wobei das eine der beiden Rohre dazu dient, als Kabelschacht die zwischen den einzetnen Komponenten der vom Stationswagen 1 aufgenommenen Rechneranlage verlegten Verbindungskabel aufzunehmen. Das andere der beiden Rohre dient in der schon vorbeschriebenen Weise der Aufnahme einer elektromotorischen Verstelleinrichtung zur teleskopierbaren Verstellung der Säule 4. Mittels der Verbinder 23 wird indes eine zusätzliche Aussteifung erreicht, so dass eine insgesamt sehr tragfähige und insbesondere gegenüber Biegebeanspruchungen unempfindliche Säule 4 ausgebildet ist.

Für eine bevorzugterweise geräuscharme Verfahrbewegung der einzelnen Segmente 17, 18, 19 der Säule 4 sind die jeweils verfahrbar ausgebildeten Segmente unter jeweiliger Zwischenordnung einer Hülse 25 in dem zugeordneten Segment untergebracht. Die Hülsen 25 bestehen bevorzugter Weise aus Kunststoff, wohingegen die Säule 4 bzw. deren Segmente 17, 18 und 19 vorzugsweise aus Metall, beispielsweise Aluminium gebildet sind. Durch die Zwischenordnung der Hülsen 25 wird ein direkter Metallkontakt vermieden.

Das Schrankoberteil 3 verfügt in der schon vorbeschriebenen Weise über einen Auszug 14, der in Verfahrrichtung 34 verfahrbar ist, wie sich dies insbesondere aus der Darstellung nach Fig. 3 ergibt.

Der Auszug 14 stellt eine Platte 26 bereit, die bedienerseitig eine mit einem Griff 28 ausgerüstete Blende 27 trägt. Die Platte 26 des Auszugs 14 dient im gezeigten Ausführungsbeispiel der Aufnahme einer Tastatur 29 zur Bedienung einer Rechneranlage.

Der Auszug 14 verfügt des Weiteren über ein Plattenelement 30. Dieses ist in Bezug auf die Höhenrichtung 7 unterhalb der Platte 26 ausgebildet und ist quer zur Verfahrrichtung 34 des Auszugs 14 verfahrbar ausgebildet. Im gezeigten Ausführungsbeispiel sind die Verfahrbewegung 34 des Auszugs 14 und die Verfahrbewegung 35 des Plattenelements 30 senkrecht, d.h. orthogonal zueinander ausgerichtet.

Das Plattenelement 30 dient im gezeigten Ausführungsbeispiel als Mauspad und nimmt im bestimmungsgemäßen Verwendungsfall in entsprechender Weise eine Maus zur Bedienung der vom Stationswagen 1 bereitgestellten Rechneranlage auf.

Das Plattenelement 30 verfügt über einen umlaufenden Kragen 31. Dieser greift in von Haltestegen 32 bereitgestellte Führungsnuten 33 des Auszugs 14 ein. Dabei wirken der Kragen 31 und die zugehörigen Führungsnuten 33 nach Art eines Schienen-Systems zusammen, womit eine geführte Verfahrbewegung des Plattenelements 30 relativ gegenüber dem Auszug 14 gewährleistet ist.

Es ist vorgesehen, dass das Plattenelement 30 mit Blickrichtung auf den Stationswagen 1 wahlweise entweder nach links oder nach rechts verfahren werden kann. Fig. 3 zeigt indes eine Ausrichtung, dergemäß das Plattenelement 30 nach rechts verfahren ist.

Die Möglichkeit das Plattenelement 30 wahlweise nach links oder nach rechts zu verfahren erbringt den Vorteil, dass eine Inverwendungnahme des Stationswagens 1 gleichermaßen für Linkshänder als auch für Rechtshänder gestattet ist.

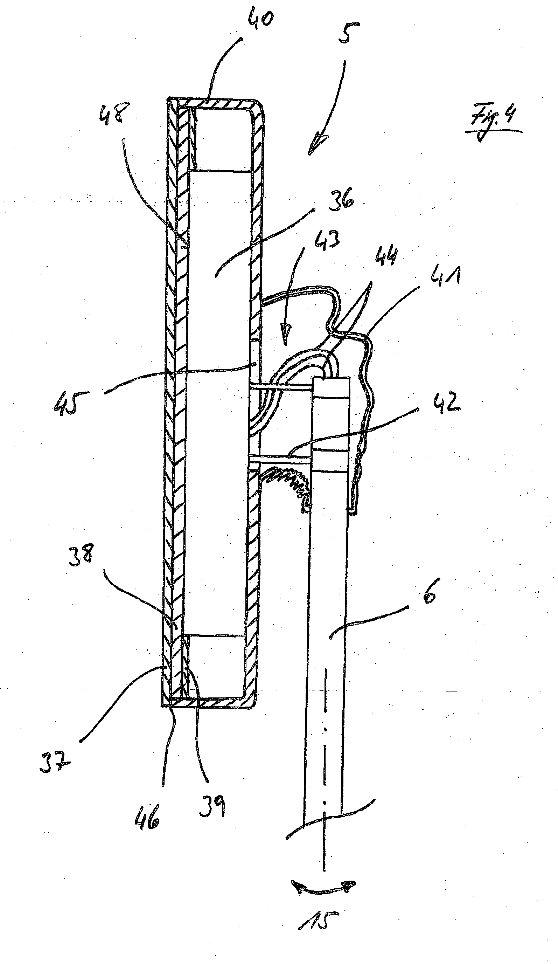

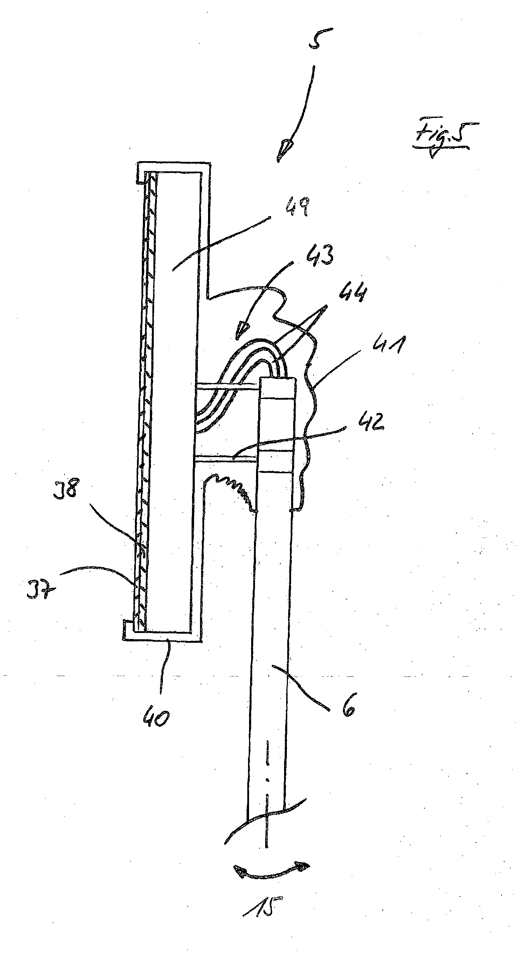

Der Monitor 5 des erfindungsgemäßen Stationswagens 1 ist aus Gründen der vereinfachten Bedienung mit Touch-Funktion ausgerüstet und insbesondere aus Hygienegründen vollgekapselt ausgebildet. Zwei alternative Ausgestaltungsformen des Monitors 5 sind in den Figuren 4 und 5 dargestellt, wobei die Ausführungsform nach Fig. 4 die bevorzugte Ausführungsförm zeigt.

Der Monitor 5 nach Fig. 4 verfügt über eine Monitoreinheit 36. Bei dieser Monitoreinheit 36 handelt es sich um einen Standardmonitor ohne Touch-Funktion. Diese Monitoreinheit 36 ist unter Zwischenordnung eines Traggestells 42 am Monitorhalter 6 angeordnet, welcher Monitorhalter 6 im gezeigten Ausführungsbeispiel als Tragrohr ausgebildet ist.

Die Monitoreinheit 36 stellt benutzerseitig eine Fläche 48 bereit. Mit dieser Fläche 48 schließt frontseitig bündig ein Rahmen 39 ab, der die Monitoreinheit 36 aufnimmt.

Frontseitig der Monitoreinheit 36 und des die Monitoreinheit 36 umgebenden Rahmens 39 ist eine Abdeckplatte 37 angeordnet. Diese Abdeckplatte 37 ist bevorzugterweise aus Glas gebildet und schützt die Monitoreinheit 36.

Zwischen der Monitoreinheit 36 und dem Rahmen 39 einerseits sowie der Abdeckplatte 37 andererseits ist eine als Touchscreen dienende Folienscheibe 38 angeordnet. Diese überragt im gezeigten Ausführungsbeispiel die fröntseitige Fläche 48 der Monitoreinheit 36 und ist in dem die Fläche 48 überragenden Bereich durch den die Monitoreinheit 36 umgebenden Rahmen 39 abgestützt. Aus fertigungstechnischen Gründen ist es bevorzugt, die Folienscheibe 38 vollflächig mit der Abdeckplatte 37 zu verkleben.

Der Monitor 5 verfügt ferner über ein Gehäuse 40. Zur Anbindung der Monitoreinheit 36 an das Traggestell 42 verfügt das Gehäuse 40 auf seiner Rückseite über einen Durchbruch 45. Dieser Durchbruch 45 dient ebenfalls dazu, Kabel 44 bzw. einen mehrere Kabel 44 umfassenden Kabelstrang 43 abzuführen. Diese sind in einen vom Tragrohr 6 bereitgestellten Kabelkanal eingeführt und in der schon vorbeschriebenen Weise zu der vom Schrankunterteil 2 des Stationswagens 1 beherbergten Rechneranlage geführt.

Wie Fig. 4 erkennen lässt, verfügt das Gehäuse 40 tragrohrseitig über eine Manschette 41. Diese deckt den monitorseitigen Endabschnitt des Tragrohrs 6, den vom Gehäuse 40 bereitgestellten Durchbruch 45 sowie die von der Monitoreinheit 36 abgehenden und in den vom Tragrohr 6 bereitgestellten Kabelkanal einmündenden Kabel 44 vollständig ab.

Die Abdeckplatte 37 schließt das Gehäuse 40 frontseitig fugenfrei ab. Es ist zu diesem Zweck konstruktiv vorgesehen, dass die Abdeckplatte 37 in ihrer Größenabmessung etwas größer als die dahinter liegende Folienscheibe 38 bzw. der dahinter liegende Rahmen 39 ausgebildet ist. Es entsteht auf diese Weise ein umlaufender Absatz 46. Dieser Absatz 46 ist in Entsprechung der Wandungsdicke des Gehäuses 40 ausgebildet, so dass ein allseits bündiger Abschluss des Gehäuses 40 an der Abdeckplatte 37 gestattet ist.

Eine alternative Ausgestaltung des Monitors 5 ist in Fig. 5 gezeigt.

Der Monitor 5 verfügt über ein Touchscreen 49, d. h. eine Monitoreinheit 36 mit Touchscreen-Funktion. Hierbei handelt es sich um ein aus dem Stand der Technik an sich bekanntes, standardisiertes Bauteil.

Der Monitor 5 verfügt ferner über eine Abdeckplatte 37, die im gezeigten Ausführungsbeispiel als Glasscheibe ausgebildet ist. Diese ist frontseitig des Touchscreens 49 angeordnet und schützt mithin die Bildschirmoberfläche des Touchscreens 49 insbesondere vor Verschmutzungen. Eine Bedienung des Touchscreens 49 erfolgt mithin unter Zwischenschaltung der Abdeckplatte 37, wobei über die Abdeckplatte 37 durch benutzerseitige Berührung eingegebene Befehle auf den Touchscreen 49 übertragen werden, welcher dann in an sich bekannter Weise eine Verarbeitung der getätigten Eingabebefehle vornimmt.

Zur Eingabeübertragung von der Abdeckplatte 37 auf den Touchscreen 49 dient eine Eingabeübertragungseinheit 38, die im gezeigten Ausführungsbeispiel als kapazitive Folienscheibe ausgebildet ist. Diese ist im Spalt zwischen der vom Touchscreen 49 beabstandet angeordneten Abdeckplatte 37 einerseits und dem Touchscreen 49 andererseits angeordnet. Der Touchscreen 49 ist im Übrigen von einem Gehäuse 40 umgeben. Es ist so insgesamt eine voll gekapselte Ausgestaltung des Monitors 5 geschaffen, womit dieser vor ungewollten Einflüssen, insbesondere durch Verschmutzungen geschützt ist. Im Verschmutzungsfall ist eine Reinigung in einfacher Weise möglich, wobei die Materialien für die Abdeckplatte 37 einerseits und das Gehäuse 40 andererseits derart gewahlt sind, dass eine bestimmungsgemäße Reinigung mit im Krankenhausbereich herkömmlichen Reinigungsmitteln vorgenommen werden kann.

Zur Anordnung des Monitors 5 an den Stationswagen dient das Tragrohr 6. Unter Zwischenordnung eines Traggestells 42 ist der Monitor 5 an dem Tragrohr 6 befestigt, wobei bevorzugterweise eine verdrehbar Anordnung in Richtung des Pfeils 15 um die vom Tragrohr 6 bereitgestellte Mittelachse gestattet ist. Es kann darüber hinaus eine Verdrehbewegung des Monitors 5 möglich sein, die eine Verschwenkung des Monitors 5 aus einer Horizontalausrichtung in eine Senkrechtausrichtung gestattet, welche Verstellmöglichkeit in den Fign. nicht im Besonderen dargestellt ist.

Das Tragrohr 6 stellt einen Kabelkanal zur Verfügung, der der Aufnahme eines mehrere Kabel 44 umfassenden Kabelstrangs 43 dient; der vom Monitor 5 abgeht. Das Gehäuse 40 verfügt tragsäulenseitig über eine Manschette 41, die den monitorseitigen Endabschnitt des Tragrohrs 6 und damit insbesondere auch den Bereich der Einführung der Kabel 44 in den vom Tragrohr 6 bereitgestellten Kabelkanal abdeckt. Es wird so eine leicht zu reinigende und insofern aus hygienischen Gründen vorteilhafte Anordnung des Monitors 5 an der Tragsäule 6 erreicht.

**Bezugszeichenliste**

| | | | |
|---|---|---|---|
| 1 | Stationswagen | 30 | Plattenelement |
| 2 | Schrankunterteil | 31 | Kragen |
| 3 | Schrankoberteil | 32 | Haltesteg |
| 4 | Säule | 33 | Führungsnut |
| 5 | Monitor | 34 | Verfahrrichtung Auszug |
| 6 | Monitorhalter | 35 | Verfahrrichtung Plattenelement |
| 7 | Höhenrichtung | 36 | Monitoreinheit |
| 8 | Pfosten | 37 | Abdeckplatte |
| 9 | Tragrolle | 38 | Folienscheibe |
| 10 | Rammschutz | 39 | Rahmen |
| 11 | Deckplatte | 40 | Gehäuse |
| 12 | Kastenteil | 41 | Manschette |
| 13 | Ablagefläche | 42 | Trag gestell |
| 14 | Auszug | 43 | Kabelstrang |
| 15 | Pfeil | 44 | Kabeln |
| 16 | Tür | 45 | Durchbruch |
| 17 | unteres Segment | 46 | Absatz |
| 18 | mittlere Segment | 47 | Schrankteil |
| 19 | oberes Segment | 48 | Fläche |
| 20 | Bedieneinheit | 49 | Touchscreen |
| 21 | erstes Rohr | | |
| 22 | zweites Rohr | | |
| 23 | Verbinder | | |
| 24 | Schraube | | |
| 25 | Hülse | | |
| 26 | Platte | | |
| 27 | Blende | | |
| 28 | Griff | | |
| 29 | Tastatur | | |

## Patentansprüche

1. Stationswagen, insbesondere Visiten- und Pflegewagen für Krankenhäuser, Pflegeheim und sonstige Einrichtungen des Gesundheitswesens, mit einem fahrbar ausgebildeten Schrankteil (47), das zur Aufnahme unter anderem einer Rechneranlage dient, und mit einem Monitor (5), der verdrehbar an einem Tragrohr (6) angeordnet ist, aufweisend, einen Touchscreen, der vollständig gekapselt ausgebildet ist und frontseitig eine aus einem transparenten Material gebildete Abdeckplatte (37) trägt, **dadurch gekennzeichnet, dass** das Schrankteil (47) ein Schrankunterteil (2) und ein Schrankoberteil (3) aufweist, wobei das Schrankoberteil (3) in Höhenrichtung (7) relativ verfahrbar zum Schrankunterteil (2) ausgebildet ist, wobei eine das Schrankoberteil (3) tragende Säule (4) vorgesehen ist, die zwei zueinander nebengeordnete, teleskopierbare Einzelrohre (21, 22), aufweist, wobei das eine der beiden Einzelrohre (21, 22) einen Kabelkanal bereitstellt und das andere der beiden Einzelrohre (21, 22) einen elektromotorischen Antrieb aufnimmt.

2. Stationswagen nach Anspruch 1, **dadurch gekennzeichnet**, das die Einzelrohre (21, 22) miteinander verbunden sind.

3. Stationswagen nach Anspruch 2, **gekennzeichnet durch** mit den Einzelrohren (21, 22) verschraubte Verbinder (23).

4. Stationswagen nach einem der vorhergehenden Ansprüche 1 bis 3, **dadurch gekennzeichnet, dass** das Schrankoberteil (3) einen nach Art einer Schublade aus dem Schrankoberteil (3) heraus verfahrbaren Auszug (14) bereitstellt, der seinerseits eine quer, vorzugsweise orthogonal zur Verfahrrichtung (34) des Auszugs (14) verfahrbares Plattenelement (30) aufweist.

5. Stationswagen nach Anspruch 4, **dadurch gekennzeichnet, dass** das Plattenelement (30) wahlweise zur einen oder zur anderen Seite des Auszugs (14) verfahrbar ist.

## Claims

1. A trolley, in particular ward trolley and care trolley for hospitals, nursing homes and other health care establishments, comprising a movable cabinet element (47) which serves to receive, inter alia, a computer system, and comprising a monitor (5) which is arranged in a rotatable manner on a carrier tube (6), comprising a touch screen which is completely encapsulated and carries a cover plate (37) made of a transparent material on the front side, **characterized in that** the cabinet element (47) comprises a cabinet bottom part (2) and a cabinet top part (3), wherein the cabinet top part (3) can be displaced with respect to the cabinet bottom part (2) in the direction of height (7), wherein a column (4) which carries the cabinet top part (3) is provided, which column comprises two individual tubes (21, 22) which are arranged side by side and which can be telescoped, wherein the one of the two individual tubes (21, 22) provides a cable channel and the other one of the two individual tubes (21, 22) receives an electromotive drive.

2. A trolley according to claim 1, **characterized in that** the individual tubes (21, 22) are connected to each other.

3. A trolley according to claim 2, **characterized by** connectors (23) screwed with the individual tubes (21, 22).

4. A trolley according to one of the preceding claims 1 through 3, **characterized in that** the cabinet top part (3) provides an extractable element (14) which can be moved out of the cabinet top part (3) like a drawer, which extractable element itself comprises a plate element (30) which can be displaced transversely, preferably in an orthogonal direction with respect to the direction of movement (34) of the extractable element (14).

5. A trolley according to claim 4, **characterized in that** the plate element (30) can be selectively displaced towards the one side or the other side of the extractable element (14).

## Revendications

1. Chariot, notamment chariot de visites et de soins, destiné à des hôpitaux, des établissements de soins ou à d'autres établissements de santé, comprenant un élément d'armoire mobile (47) qui sert à recevoir, entre autres, un système d'ordinateur, et comprenant un moniteur (5) qui est disposé sur un tube de support (6) de manière rotative, comprenant un écran tactile qui est complètement encapsulé et qui porte sur la face avant une plaque de couverture (37) en matière transparente, **caractérisé en ce que** l'élément d'armoire (47) comprend une partie d'armoire inférieure (2) et une partie d'armoire supérieure (3), la partie d'armoire supérieure (3) étant susceptible d'être déplacée dans la direction de hauteur par rapport à la partie d'armoire inférieure (2), une colonne (4) portant la partie d'armoire supérieure (3) étant prévue, laquelle colonne comprend deux tubes individuels (21, 22) qui sont disposés l'un à côté de l'autre et qui sont télescopiques, l'un des deux tubes individuels (21, 22) fournissant un canal de câbles et l'autre des deux tubes individuels (21, 22) recevant un entraînement à moteur électrique.

2. Chariot selon la revendication 1, **caractérisé en ce que** les tubes individuels (21, 22) sont reliés l'un à l'autre.

3. Chariot selon la revendication 2, **caractérisé par** des connecteurs (23) qui sont vissés avec les tubes individuels (21, 22).

4. Chariot selon l'une des revendications 1 à 3, **caractérisé en ce que** la partie d'armoire supérieure (3) fournit un élément d'extension (14) qu'on peut sortir de la partie d'armoire supérieure (3) à la façon d'un tiroir, l'élément d'extraction lui-même comprenant un élément de plaque (30) qui est déplaçable transversalement, de préférence orthogonalement par rapport à la direction de déplacement (34) de l'élément d'extraction (14).

5. Chariot selon la revendication 4, **caractérisé en ce que** l'élément de plaque (35) peut sélectivement être déplacé vers l'un côté ou vers l'autre côté de l'élément d'extraction (14).
